(19) Europäisches Patentamt / European Patent Office / Office européen des brevets

(11) **EP 2 870 911 B1**

(12) **FASCICULE DE BREVET EUROPEEN**

(45) Date de publication et mention
de la délivrance du brevet:
**10.04.2019 Bulletin 2019/15**

(51) Int Cl.:
*A61B 5/00* *(2006.01)*     *G01C 5/06* *(2006.01)*
*A61B 5/11* *(2006.01)*     *A63B 24/00* *(2006.01)*
*G01C 22/00* *(2006.01)*     *G01C 5/00* *(2006.01)*

(21) Numéro de dépôt: **14192074.4**

(22) Date de dépôt: **06.11.2014**

(54) **Procédé d'estimation de dénivelé parcouru par un utilisateur**

Verfahren zur Schätzung der zurückgelegten Höhenunterschiede eines Benutzers

Method for estimating the elevation travelled by a user

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.11.2013 FR 1360909**

(43) Date de publication de la demande:
**13.05.2015 Bulletin 2015/20**

(73) Titulaire: **Withings**
**92130 Issy-les-Moulineaux (FR)**

(72) Inventeurs:
• **Thomas, Rémi**
**92170 Vanves (FR)**
• **Carreel, Eric**
**92190 Meudon (FR)**

(74) Mandataire: **Cabinet Plasseraud**
**66, rue de la Chaussée d'Antin**
**75440 Paris Cedex 09 (FR)**

(56) Documents cités:
**EP-A1- 2 083 244**     **WO-A1-2013/136251**
**DE-A1-102008 049 750**     **US-A- 5 058 427**
**US-A- 5 295 085**

**Description**

La présente invention est relative aux procédés d'estimation de dénivelé parcouru par un utilisateur.

Plus particulièrement, l'invention se rapporte à un procédé d'estimation du dénivelé franchi par un utilisateur, au moyen d'un dispositif destiné à être porté par l'utilisateur, ledit dispositif étant adapté pour mesurer la pression ambiante et en déduire l'altitude correspondante. La mesure des changements de pression permet d'en déduire le changement d'altitude correspondant et par conséquent d'en déduire un dénivelé franchi par l'utilisateur.

Le document US5058427 divulgue un calcul des dénivelés positifs pour un cycliste. Ce document ne divulgue pas un calcul de variance des valeurs de pression afin de détecter les dénivelés positifs.

Le document US2012084053 décrit un dispositif de surveillance et d'enregistrement des mouvements d'un utilisateur et il s'attache en particulier à déterminer des dénivelés franchis par cet utilisateur. Cependant le procédé d'estimation mis en oeuvre dans ce document utilise un algorithme à base de filtres à seuil et de mécanismes d'hystérésis.

Un tel algorithme peut être perturbé par des valeurs ponctuelles aberrantes qui peuvent conduire à des déclenchements indésirables du comptage, notamment si le dispositif qui comprend le capteur de pression est soumis à des phénomènes acoustiques qui peuvent se produire lorsque l'individu rentre dans une pièce, ouvre une porte, prend l'ascenseur, se trouvent à bord d'un véhicule qui rentre et sort d'un tunnel. De plus, un tel algorithme requiert une capacité de calcul et une capacité de mémoire significatives.

Il est donc apparu un besoin de proposer un procédé de filtrage amélioré des valeurs de pression mesurées ou converties en valeurs d'altitude, de manière à pouvoir éliminer les valeurs aberrantes, tout en donnant les dénivelés parcourus en quasi-temps réel, un tel procédé ne devant pas nécessiter une trop grande capacité de calcul.

Dans l'ensemble de la demande on désignera par le terme « buffer » une mémoire tampon.
A cet effet, l'invention propose un procédé selon la revendication 1 et un dispositif selon la revendication 13.

Grâce à ces dispositions, une telle condition de prise en compte des dénivelés basée sur le calcul de variance permet d'éliminer des valeurs potentiellement aberrantes ; les étapes d'échantillonnage-filtrage-calcul de variance permettent d'obtenir une estimation fiable en quasi temps réel du dénivelé à partir de valeurs réellement mesurées, et en même temps d'éliminer d'éventuelles valeurs de pression aberrantes ou correspondant à un phénomène acoustique perturbateur. De plus l'étape de calcul de variance est un critère optimal pour discriminer un vrai mouvement de montée ou de descente par rapport à des perturbations possibles des signaux de pression. En outre, le procédé proposé et le consommateur en termes de puissance de calcul et de l'espace mémoire, ce qui est avantageux pour un dispositif de très petite taille embarqué.
Dans des modes de réalisation préférés de l'invention, on peut éventuellement avoir recours en outre à l'une et/ou à l'autre des dispositions suivantes :

- l'opération de filtrage est réalisée à la cadence de la première fréquence d'échantillonnage. *De sorte que l'opération de filtrage est synchrone avec l'opération d'échantillonnage.*
- le calcul de variance est effectué à **une deuxième** fréquence, inférieure à la première. *De sorte que la charge de calcul impliquée est relativement limitée et de plus cela espace la fréquence des calculs de variance ce qui limite le taux de faux déclenchements.*
- la première fréquence est un multiple de la deuxième fréquence. *Moyennant quoi l'horloge de la deuxième fréquence peut être obtenue facilement à partir de l'horloge de base de première fréquence.*
- le calcul de variance est réalisé sur **des** valeurs (P valeurs) filtrées sous-échantillonnées, stockées dans un second buffer. *Ainsi on travaille sur un nombre de valeurs limitées pour le calcul le plus difficile à réaliser à savoir le calcul de variance.*
- **le procédé** comprend en outre l'étape suivante : on compare le résultat du calcul de variance VAR à une **valeur seuil S** prédéterminée.

  - si VAR > S alors la **condition de comptage** est **'vraie'** et les dénivelés constatés sont pris en compte par le calcul d'estimation de dénivelé
  - si VAR < S alors la condition de comptage est **'fausse'** et les dénivelés constatés ne sont pas pris en compte par le calcul d'estimation de dénivelé.
    *Moyennant quoi on déclenche la prise en compte des dénivelés uniquement à bon escient, et on élimine les possibles bruit entachant les signaux de pression.*

- lorsque la condition de comptage passe de **'fausse'** à 'vraie', le dénivelé pris en compte se base sur la valeur la plus ancienne stockée dans le second buffer. *De sorte que nonobstant la présence du filtre qui introduit un retard, on ne perd toutefois pas substantiellement de dénivelé dans le calcul.*

L'invention vise en outre un d**ispositif** adapté pour mettre en oeuvre le procédé. L'invention vise en outre également un dispositif qui prend la forme d'un boitier moniteur d'activité personnel.

D'autres caractéristiques et avantages de l'invention apparaîtront au cours de la description suivante d'une de ses formes de réalisation, donnée à titre d'exemple non limitatif, en regard des dessins joints.

Sur les dessins :

- la figure 1 représente un individu portant un dispositif mettant en oeuvre le procédé selon l'invention,
- la figure 2 représente un moniteur d'activité personnel, comme étant forme de dispositif pouvant mettre en oeuvre le procédé selon l'invention,
- la figure 3 représente un schéma de principe d'un dispositif mettant en oeuvre le procédé selon l'invention,
- la figure 4 illustre un filtre médian,
- la figure 5 représente schématiquement les deux buffers mémoire mis en jeu dans le procédé,
- la figure 6 illustre un chronogramme des signaux d'altitude bruts et filtrés et de la variance,
- la figure 7 illustre schématiquement le déroulement du procédé.

Sur les différentes figures, les mêmes références désignent des éléments identiques ou similaires.

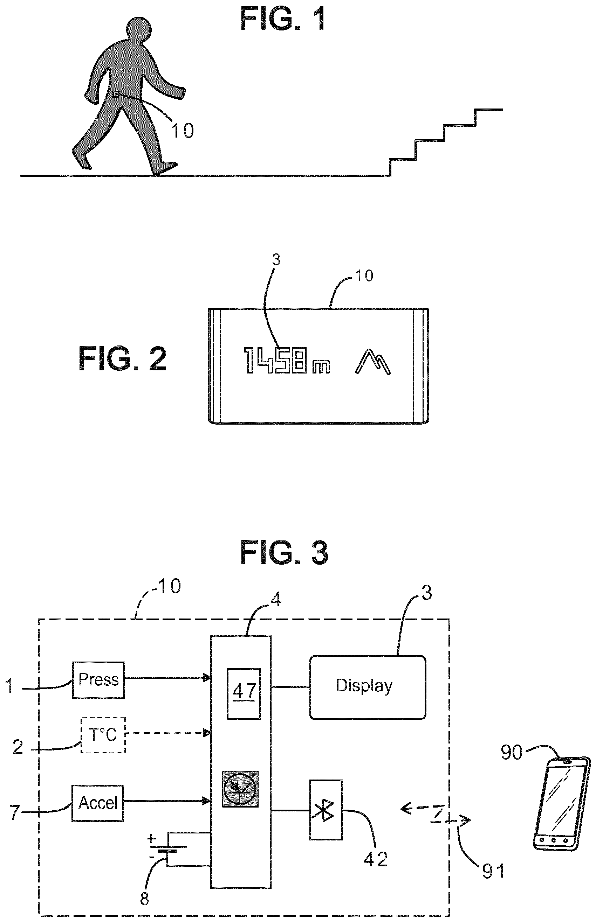

Sur la figure 1, un utilisateur porte un dispositif 10 destiné à lui indiquer les dénivelés franchis. Le dispositif 10 pourra détecter les dénivelés qu'il aura parcouru en portant ce dispositif 10. Dans l'exemple illustré à la figure 2, le dispositif 10 prend la forme d'un moniteur d'activité personnel capable de renseigner l'utilisateur sur son activité physique, marche à pied, course, sommeil, montée de marches, etc. Le dispositif 10 estimateur de dénivelé se présente par exemple sous la forme d'un boitier comportant un système d'affichage, qui permet d'afficher le dénivelé parcouru par l'utilisateur, ledit dénivelé étant déterminé par le procédé mis en oeuvre par le dispositif 10. Selon une variante non représentée, le dispositif 10 peut former partie intégrante d'un appareil téléphonique intelligent dit aussi Smartphone.

Le dispositif 10 dont le principe est illustré à la figure 3 comporte au moins un capteur de pression **1** et un capteur de température **2.** Le capteur de pression 1 est adapté pour fournir des valeurs de pression. Le capteur de température est adapté pour fournir des valeurs de température afin de compenser les valeurs de pression. Les valeurs de pression mesurées peuvent par exemple être corrélées via un abaque aux valeurs de température constatées, et corrigées en conséquence.

Le dispositif 10 (et en particulier le capteur de pression contenu) est adapté pour être maintenu sensiblement solidaire à au moins une partie du corps dudit utilisateur au cours de la réalisation dudit procédé, par exemple porté à la ceinture de l'utilisateur (cf Fig. 1), ou sur un bracelet fixé au poignet de l'utilisateur. Il peut être encore intégré dans un appareil de type téléphone intelligent porté par l'utilisateur, voire encore dans une oreillette.

Le dispositif 10 comprend une unité de calcul 4 à laquelle sont raccordés les capteurs de température 1 et pression 2. Il est également prévu un capteur d'accélération 7 multi-axes pour détecter des accélérations subies par le dispositif, à partir desquelles on estime les accélérations subies par l'utilisateur. Les informations d'accélération peuvent être utilisées pour différencier les dénivelés réellement liés à une ascension de ceux liés à une ascension mécanique telle qu'avec un ascenseur par exemple.

L'unité centrale 4 comprend un afficheur 3 (aussi appelé display) configuré pour mettre à disposition de l'utilisateur une pluralité d'information et notamment un compteur de dénivelé positif parcouru (dénivelé montant). La remise à zéro de ce compteur peut être journalière ou autre suivant la configuration donnée par l'utilisateur.

L'unité centrale 4 traite les valeurs via un processeur qui comprend une zone mémoire **47** avec des buffers dont l'utilité sera précisée plus loin.

L'unité centrale 4 commande système d'affichage 3 et/ou communique via un une interface de communication Bluetooth™ 42 avec un smartphone 90, via une interaction de valeurs **91.** Un affichage complémentaire peut se faire sur le smartphone 90 et/ou la gestion de configuration par une application smartphone liée au moniteur d'activité.

L'unité centrale 4 est alimentée par une source d'énergie embarquée **8,** ici par exemple une batterie rechargeable. Cette batterie alimente tous les éléments embarqués dans le dispositif, à savoir les capteurs 1, 2, 7, le display 3 et l'unité centrale 4.

Le procédé pourra être réalisé soit à partir des valeurs de pression soit après conversion des valeurs de pression en valeurs d'altitude. Cette conversion pourra être faite à une étape quelconque du procédé en utilisant la relation :

$$p(z) = 1013.25 * \left(1 - \frac{0.0065 * z}{288.15}\right)^{5.255}$$

**p** désignant la pression atmosphérique,
**z** désignant l'altitude par rapport au niveau moyen de la mer. On voit ici que selon la relation biunivoque entre altitude et pression, on pourra travailler soit en altitude soit en pression indifféremment.

Dans un mode de réalisation privilégié de l'invention, la conversion de valeurs de pression à valeurs d'altitude aura lieu dès le prélèvement des valeurs et ce sont les données brutes d'altitude et non de pression qui seront stockées les buffers mémoires.

Dans toute la suite on considèrera le cas le plus général de valeurs qu'elles soient de pression ou d'altitude.

Dans un mode de réalisation privilégié de l'invention, le procédé est mis en oeuvre selon les étapes suivantes :
Une opération d'échantillonnage périodique des valeurs brutes est réalisée. Les valeurs brutes sont relevées avec une première fréquence d'échantillonnage **F1,** puis stockées dans un premier buffer **B1.** Ledit premier buffer B1 comprend par exemple **N** cases mémoire. La première fréquence F1 peut être par exemple de 1Hz (donc un top d'horloge toutes les secondes).

Les valeurs brutes sont en permanence renouvelées par de nouvelles valeurs qui sont échantillonnées au cours du temps. A chaque nouvelle valeur échantillonnée, elle est placée dans la dernière case mémoire remplie soit la Nième, les autres valeurs sont décalées vers la première case mémoire, et la valeur qui était comprise dans la première case remplie est perdue pour être donc remplacée par celle qui se trouvait dans la seconde case remplie, juste avant la dernière acquisition (principe de la fenêtre glissante).

A cette même première fréquence F1, qui correspond à des tics d'horloge espacés d'une période T1 avec T1=1/F1, un filtre est appliqué à l'ensemble des valeurs contenues dans le premier buffer B1. C'est-à-dire qu'à chaque tic d'horloge un filtre est appliqué à l'ensemble des valeurs contenues dans le premier buffer B1 à cet instant. Il peut par exemple s'agir d'un filtre médian FM qui prend la médiane de l'ensemble des valeurs contenues dans le premier buffer B1 à l'instant de l'application du filtre. Il peut également s'agir d'un autre type de filtre.

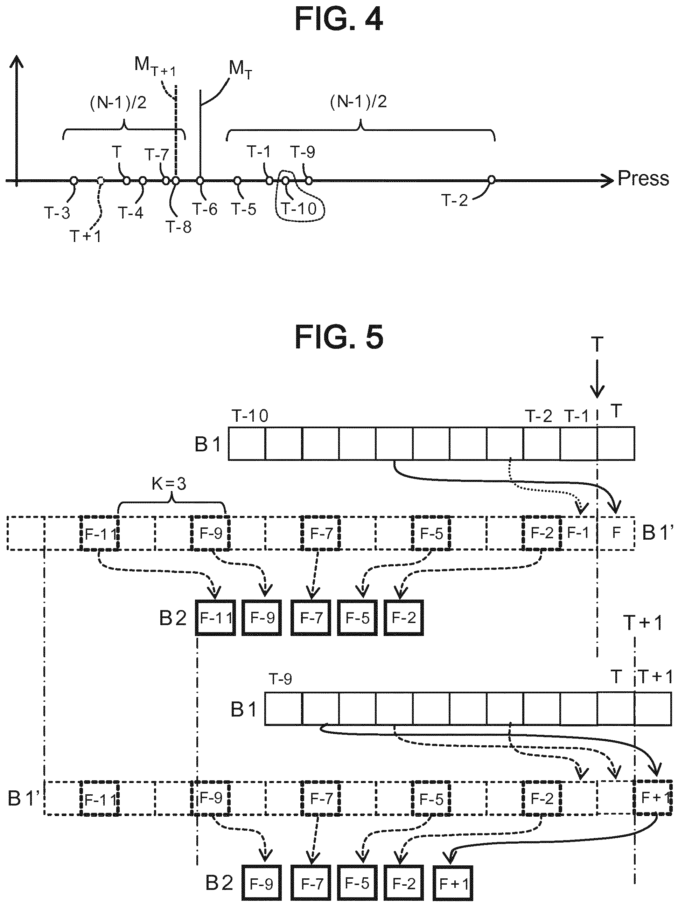

Le cas d'un filtre médian **FM** est illustré à la figure 4. Lorsqu'il est appliqué aux N valeurs stockées dans le premier buffer B1, avec N impair, les N valeurs sont ordonnées et est retenue comme la médiane la valeur réellement mesurée qui comprend autant de valeurs inférieures (N-1)/2 que de valeurs supérieures (N-1)/2. Le filtre médian FM peut également être appliqué à un nombre pair de valeurs. Dans ce cas, l'ensemble de N valeurs est divisé en deux sous ensemble de N/2 valeurs et la valeur médiane est l'une des valeurs réellement mesurée entre la valeur maximale du sous ensemble inférieur et la valeur minimale du sous ensemble supérieur.

L'utilisation d'un filtre médian FM implique notamment un retard dans l'observation d'un dénivelé. En effet lorsque les valeurs brutes relevées vont avoir une tendance à se décaler vers des valeurs plus élevées par exemple, plusieurs nouvelles acquisitions seront nécessaires avant que la valeur médiane des valeurs se trouve d'autant décalée. L'utilisation d'un filtre médian FM permet l'élimination de valeur(s) aberrante(s) résultant d'un phénomène acoustique bref.

Les valeurs ainsi filtrées sont ensuite sous échantillonnées à la deuxième fréquence F2, de préférence multiple de la première fréquence F1, et les valeurs sélectionnées par le sous échantillonnage sont stockées dans un second buffer **B2.**

Un calcul de variance est alors effectué à partir de ces valeurs filtrées sous échantillonnées. Le calcul de variance utilise donc au moins une partie des valeurs filtrées. Le sous échantillonnage permet de limiter le nombre de valeurs utilisées pour le calcul de variance, et ainsi de limiter le besoin de puissance de calcul.

Le second buffer **B2** peut contenir plus ou moins de valeurs que le premier buffer B1. Considérons que le second buffer B2 comprend P disponibilités. Les valeurs sous échantillonnées stockées au nombre de **P** dans le second buffer B2 sont donc renouvelées régulièrement. A chaque nouvelle valeur sous échantillonnée à partir du premier buffer B1, ladite nouvelle valeur est intégrée dans le second buffer B2, l'ensemble des valeurs comprises dans le second buffer B2 sont décalées vers la première valeur stockée dans le second buffer B2, et la valeur la plus anciennement stockée dans le second buffer B2 est éliminée.

La variance est donnée par la relation suivante :

$$ \text{VAR} = \frac{1}{P-1} \sum_{i=1}^{P} (xi - \overline{x})^2 $$

où les xi sont les valeurs contenues dans le second buffer B2 et où $\overline{X}$ est la moyenne des xi,

Le calcul de variance est donc réalisé dans ce mode de réalisation sur le second buffer B2 à la deuxième fréquence F2, c'est-à-dire à chaque fois que le second buffer B2 est mis à jour. La deuxième fréquence F2 correspond à une horloge dont les tics seraient espacés d'une période T2. Le calcul de variance est donc réalisé à partir des valeurs stockées dans le second buffer B2 à l'instant du tic.

Le facteur multiplicatif entre la première fréquence F1 et la deuxième fréquence F2 peut par exemple être choisi par l'utilisateur.

Le retard lié au filtrage des valeurs brutes se répercute donc sur la valeur de la variance qui est calculée à partir de valeurs filtrées sous échantillonnées. La profondeur de stockage du second buffer B2 est adaptée pour permettre la mise en mémoire de suffisamment de valeurs échantillonnées pour pouvoir calculer ultérieurement le dénivelé.

Le calcul de la variance permet dans le cas le plus général de définir une condition de prise en compte ou non des dénivelés correspondants aux différences de pression constatées.

La variance est utilisée pour déterminer si un vrai dénivelé a été franchi ou non par l'utilisateur.

La valeur de la variance VAR est comparée à un seuil S.

- Si la valeur de la variance VAR est inférieure au seuil S, alors la condition de comptage est **'fausse'** et les dénivelés constatés ne sont pas pris en compte par le calcul d'estimation de dénivelé.
- Si la valeur de la variance VAR est supérieure au seuil S, la **condition de comptage** est **'vraie'** et les dénivelés constatés sont pris en compte par le calcul d'estimation de dénivelé.

Dans le cas le plus général lorsque la variance est supérieure au seuil et qu'un dénivelé est franchi, le dénivelé parcouru est estimé en calculant la différence **D** entre les deux dernières valeurs successives filtrées échantillonnées stockées dans le buffer B2.

Une situation particulière peut être isolée pour la première fois que la variance VAR dépasse le seuil S. Le dénivelé est par exemple estimé en calculant la différence entre la dernière valeur filtrée échantillonnée stockée dans le buffer B2, et la valeur la plus ancienne qui avait été stockée dans le second buffer B2. Il peut également s'agir de la différence entre la dernière valeur filtrée échantillonnée stockée dans le second buffer B2, et une valeur plus ancienne qui avait été stockée dans le second buffer B2.

La valeur de dénivelé ainsi estimée permet de rattraper le retard de prises d'informations sur le franchissement du dénivelé lié au retard dans l'estimation de la variance par l'utilisation de valeurs filtrées échantillonnées (voir ci-dessus). Donc un dénivelé sera détecté avec un retard via la variance VAR et ce retard dans la détection pourra être compensé en évaluant le dénivelé via des valeurs dans le second buffer B2 plus anciennes. Cette correction ou compensation à postériori sera plus ou moins exacte en fonction de la profondeur du second buffer B2 et du choix fait de la seconde valeur pour effectuer la différence.

La variance VAR peut être ponctuellement au-dessus du seuil sans correspondre réellement à un changement de tendance. Cela peut aussi être corrigé par le fait que la première fois que la variance est au-dessus du seuil la différence est faite entre une ancienne valeur du second buffer B2 et la dernière valeur enregistrée dans le second buffer B2. Ainsi la différence entre ces deux valeurs éloignées aide à discriminer une variance ayant réellement franchi le seuil du cas où il s'agit d'un franchissement ponctuel.

Si la différence D est positive, le dénivelé est considéré comme une ascension et est stocké dans un premier compteur C1, pour être cumulé aux autres valeurs retenues. Cette valeur est celle qui va intéresser l'utilisateur qui souhaite obtenir une estimation de son ascension.

Si la différence D est négative, le dénivelé correspond à une descente et est stocké dans un second compteur C2. Ce second compteur C2 pourra notamment dans un mode de réalisation de l'invention permettre de vérifier la validité de l'ascension mesurée en pouvant vérifier que pour un retour à un point de départ la somme des ascensions et des descentes donne bien un dénivelé nul.

Lorsque la variance VAR repasse sous le seuil S, le dénivelé est considéré nul et n'est incrémenté dans aucun compteur.

Lors d'une brusque inversion de tendance, d'ascension vers descente par exemple, la variance VAR reste au-dessus du seuil S mais la différence D entre des valeurs successives passe d'une différence positive à une différence négative. Dans ce cas au moins trois valeurs seront relevées et les différences seront effectuées avant de valider la situation de redescente. Après validation, la redescente sera établie à partir de la première valeur initialement soupçonnée de redescente.

A l'état initial, tous les buffers sont vides. Lorsque les trois premières valeurs sont relevées par le détecteur, la médiane est calculée et l'ensemble des valeurs contenues dans le premier buffer B1 est rempli/écrasé par cette valeur. Ensuite, de nouvelles valeurs sont relevées et entrées dans le premier buffer B1 puis le procédé classique tel que décrit précédemment se poursuit. Il en est de même pour l'ensemble des valeurs du second buffer B2.

Plus précisément un cas particulier ici illustré par les figures 4 et 5 peut être décrit.

Comme illustré à la figure 4, le filtre médian FM permet de stocker les valeurs médianes de chaque sous-ensemble de valeurs considéré. Ainsi les valeurs erreurs sont supprimées. Dans la figure 4 par exemple, la valeur prise à T-2 est filtrée par le filtre médian FM. Cette valeur deviendra l'indication d'une tendance de dénivellation lorsque l'ensemble des valeurs sur lequel est appliqué le filtre médian FM se situera autour de T-2.

Concernant les étapes de filtrage et d'échantillonnage, comme dans l'exemple illustré par la figure 5, pour être plus précis le raisonnement peut être mené dans le cas d'un premier buffer B1 comprenant 11 cases mémoire. Considérons T l'instant auquel on se référera par la suite et qui correspond à un tic d'horloge. A l'instant T, les valeurs qui étaient déjà stockées dans le premier buffer B1 sont les valeurs de T-1 à T-10. A cet instant une nouvelle valeur prise à l'instant T est stockée.

Selon une première possibilité, le filtre médian FM est appliqué à la cadence F1 de cet échantillonnage de base, et dans ce cas une zone mémoire intermédiaire notée B1' est mise à jour avec le résultat de l'opération de filtrage

A chaque tic d'horloge une nouvelle valeur est issue du filtrage par exemple médian appliqué à l'ensemble des valeurs contenues dans le premier buffer B1 à l'instant du tic T soit sur les valeurs de T à T-10. La nouvelle valeur entrée dans la zone mémoire intermédiaire B1' est donc une valeur parmi les 11 valeurs comprises dans le premier buffer B1 à l'instant du tic d'horloge. Il s'agit par exemple comme illustré à la figure 5 de la valeur à T-6. Les valeurs filtrées sont notées 'F-i'.

Le second buffer B2, destiné en particulier au calcul de variance, est rempli par les valeurs filtrées sous échantillonnées, avec une fréquence d'horloge F2.

Dans le cas illustré à la figure 5, K=3 donc une valeur sur 3 est échantillonnée. A chaque valeur échantillonnée, donc toutes les trois valeurs dans l'exemple illustré, la valeur est retenue et stockée dans le buffer B2. Dans le cas illustré à la figure 5, la nouvelle valeur filtrée au tic d'horloge n'est pas la troisième donc elle n'est pas retenue lors de l'échantillonnage.

Au tic d'horloge suivant c'est-à-dire à l'instant T+1, une nouvelle valeur est entrée dans le premier buffer B1 et la valeur prise à T-10 est supprimée, pour le cas considéré dans l'exemple illustré à la figure 5. Le filtre médian FM appliqué à l'ensemble des valeurs comprises dans le premier buffer B1 à l'instant T+1 sélectionne une valeur parmi les 11 valeurs comprises dans le premier buffer B1 à l'instant T+1. Dans l'exemple illustré à la figure 5, il s'agit par exemple de la valeur retenue à T-8 dans le premier buffer B1. Dans l'exemple illustré à la figure 5, la nouvelle valeur filtrée correspond à la troisième valeur retenue selon la périodicité d'échantillonnage. La valeur filtrée à l'instant T+1 est donc stockée dans le second buffer B2 et la valeur la plus ancienne stockée dans le second buffer B2 est supprimée (principe de la fenêtre glissante).

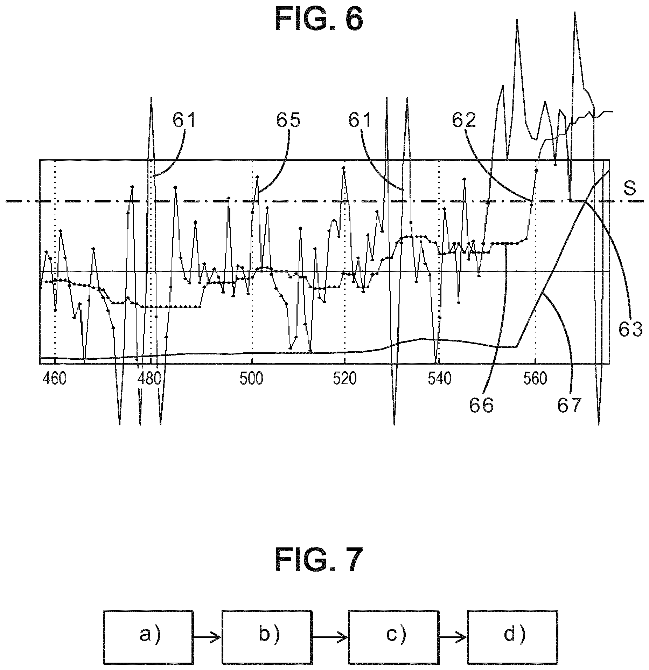

La figure 6 illustre le cumul sur un même graphique des valeurs d'altitude brutes détectées stockées dans le premier buffer B1 (courbe 65), et des valeurs d'altitude mesurées filtrées par le filtre médian FM puis stockées dans la zone mémoire B1' (courbe 66). La figure 6 illustre également la variance (courbe 67) calculée ponctuellement à partir des valeurs d'altitude filtrées, et à comparer ponctuellement au seuil **S** représenté sur le même graphique. Les valeurs aberrantes sont les valeurs repérées **61** et non pas d'influence sur le calcul de variance car elles sont éliminées par le filtre médian et le franchissement du seuil par la variance est repéré par le point **63.** On remarque que le franchissement du seuil S se produit avec un certain retard par rapport à la montée franche d'altitude autour du temps 560 (point **62**). Le choix du multiple **K** entre la première fréquence F1 et la deuxième fréquence F2 est libre au développeur. Ce choix implique cependant un compromis entre l'aspect temps-réel du résultat, et le taux de fausse alarme. Par exemple, si le développeur choisit de stocker une valeur filtrée sur deux, l'aspect temps-réel sera fort mais le taux de fausse alarme pourra être non nul quoique léger, alors que si le développeur choisit de stocker une valeur filtrée sur quatre l'aspect temps réel sera faible mais le taux de fausse alarme sera quasi-nul.

En effet la finalité de cette technique est d'extraire du signal filtré les dénivelés vrais avec le meilleur taux de détection, et un taux de fausse détection proche de 0%. La force de l'algorithme de détection proposé ici réside dans le fait que l'utilisateur peut choisir à sa convenance la pente minimum qu'il souhaite observer, ainsi que le taux de fausses détections. En effet, pour une même valeur du seuil S, plus le signal stocké est sous-échantillonné plus la pente minimum observable et le taux de fausses détections sera faible. La détermination du seuil de détection S se fait donc par rapport à une pente qui sert de minimum de référence, et du taux de fausses détections souhaité par l'utilisateur. Donc plus la distance entre les échantillons utilisés est grande, plus le seuil de détection S peut être haut, ce qui permet d'avoir un écart plus strict entre les vrais dénivelés et les faux. Mais la distance entre valeurs consécutives ne peut pas être trop élevée afin de conserver la notion de temps réel.

La figure 7 illustre l'ensemble des étapes du procédé.

Avantageusement, les valeurs aberrantes de l'ensemble des valeurs réellement mesurées sont donc supprimées par le filtrage. Mais l'utilisation d'un filtre médian FM introduit notamment un retard dans l'observation d'un dénivelé. En effet lorsque les valeurs brutes relevées vont avoir une tendance à se décaler vers des valeurs plus élevées par exemple, plusieurs nouvelles acquisitions seront nécessaires avant que la valeur médiane des valeurs se trouve d'autant décalée. Toutefois, la valeur la plus ancienne stockée dans le buffer B2 permet d'éviter la perte d'information du dénivelé réellement franchi par l'utilisateur.

Il faut noter que l'utilisation de la zone mémoire intermédiaire B1' n'est pas obligatoire ; la profondeur de la zone mémoire B1' n'est pas nécessairement la même que celle du premier buffer B1, elle peut être très faible, limité par exemple à deux ou trois positions.

## Revendications

1.  **Procédé d'estimation du dénivelé franchi par un utilisateur,** ledit procédé utilisant au moins un capteur de pression adapté pour fournir des valeurs brutes de pression et un capteur de température adapté pour fournir des valeurs de température afin de compenser les valeurs de pression, ledit **capteur de pression** étant adapté pour être maintenu sensiblement solidaire à au moins une partie du corps dudit utilisateur au cours de la réalisation dudit procédé, ledit procédé comprenant au moins :

    **a)-** une **opération d'échantillonnage** périodique des valeurs brutes de pression, relevant la pression brute courante à une première fréquence d'échantillonnage (F1), les valeurs brutes étant stockées sous forme de pression ou d'altitude correspondante dans un premier buffer (B1),
    **b)-** une opération de **filtrage** basée sur les valeurs brutes stockées dans le premier buffer (B1) pour déterminer des **valeurs filtrées,** ladite opération de filtrage étant apte à éliminer des **valeurs brutes aberrantes** qui peuvent être dues à un phénomène acoustique perturbateur,
    **c)-** un **calcul de variance (VAR),** opéré sur au moins une partie desdites valeurs filtrées, en vue de déterminer, par comparaison de ladite variance à un seuil (S), une condition de prise en compte des dénivelés correspondant à des différences de pression ou altitude constatées,
    **d)-** calculer le dénivelé franchi par l'utilisateur lors de son déplacement, dont le dénivelé cumulé positif, grâce à un **premier compteur** (C1), destiné à stocker le **cumul des dénivelés positifs** franchis, les dénivelés positifs franchis étant pris en compte si la condition de prise en compte est vraie.

2.  Procédé selon la revendication 1 pour lequel l'opération de **filtrage** utilise un filtre médian opérant sur un nombre prédéfini de N des valeurs brutes les plus récentes et l'opération de filtrage est réalisée à la cadence de la première fréquence d'échantillonnage (F1).

3. Procédé selon l'une quelconque des revendications 1 et 2 dans lequel le calcul de variance est effectué à **une deuxième** fréquence (F2), inférieure ou égale à la première.

4. Procédé selon la revendication 3, pour lequel la première fréquence (F1) est un multiple de la deuxième fréquence (F2), à savoir F1 = **k** F2.

5. Procédé selon l'une quelconque des revendications 1 à 4 pour lequel le calcul de variance est réalisé sur **P** valeurs filtrées sous-échantillonnées, stockées dans un second buffer (B2).

6. Procédé selon la revendication 3, dans lequel les valeurs filtrées sont sous échantillonnées à la deuxième fréquence (F2), et les valeurs sélectionnées par le sous échantillonnage sont stockées dans un second buffer (B2).

7. Procédé selon l'une quelconque des revendications 1 à 5 comprenant en outre l'étape suivante :

   **e)**

   - si le résultat du calcul de variance VAR > seuil S alors la **condition de comptage** est **'vraie'** et les dénivelés constatés sont pris en compte par le calcul d'estimation de dénivelé
   - si le résultat du calcul de variance VAR < seuil S alors la condition de comptage est **'fausse'** et les dénivelés constatés ne sont pas pris en compte par le calcul d'estimation de dénivelé.

8. Procédé selon la revendication 7 pour lequel lorsque la condition de comptage passe de **'fausse'** à 'vraie', le dénivelé pris en compte se base sur la valeur la plus ancienne stockée dans le second buffer (B2).

9. Procédé selon la revendication 7, dans lequel, la première fois que la variance VAR dépasse le seuil (S), le dénivelé est estimé en calculant la différence entre la dernière valeur stockée dans le second buffer (B2), et la valeur la plus ancienne stockée dans le second buffer (B2).

10. Procédé selon la revendication 1, dans lequel si les différences d'altitude constatées sont négatives, le dénivelé correspond à une descente et est stocké dans un second compteur (C2).

11. Procédé selon l'une quelconque des revendications 1 à 5, **caractérisé en ce qu'**il est capable de renseigner un utilisateur sur son activité physique incluant la marche à pied, la course, le sommeil, la montée de marches.

12. Procédé selon l'une quelconque des revendications 1 à 5, utilisant en outre un capteur d'accélération et dans lequel les informations d'accélération sont utilisées pour différencier les dénivelés réellement liés à une ascension de ceux liés à une ascension mécanique telle qu'avec un ascenseur.

13. **Dispositif** adapté pour mettre en oeuvre le procédé selon l'une quelconque des revendications 1 à 12.

14. Dispositif selon la revendication 13 qui prend la forme d'un boitier moniteur d'activité personnel capable de renseigner un utilisateur d'un tel moniteur d'activité sur son activité physique.

15. Dispositif selon la revendication 13 qui fait partie intégrante d'un smartphone.

**Patentansprüche**

1. Verfahren zum Schätzen eines von einem Benutzer überwundenen Höhenunterschieds, wobei das Verfahren wenigstens einen Drucksensor verwendet, welcher dazu eingerichtet ist, Rohwerte des Drucks zu liefern, sowie einen Temperatursensor, welcher dazu eingerichtet ist, Temperaturwerte zu liefern, um die Druckwerte anzupassen, wobei der Drucksensor dazu eingerichtet ist, im Wesentlichen befestigt an einem Abschnitt des Körpers des Benutzers während des Durchführens des Verfahrens gehalten zu werden, wobei das Verfahren wenigstens umfasst:

   a) - einen Betrieb eines periodischen Aufnehmens von Rohwerten des Drucks, wobei der momentane Rohdruck bei einer ersten Aufnahmefrequenz (F1) bestimmt wird, wobei die Rohwerte in Form eines Drucks oder einer entsprechenden Höhe in einem ersten Puffer (B1) gespeichert werden,
   b) - einen Betrieb eines Filters auf Grundlage der in dem ersten Puffer (B1) gespeicherten Rohwerte, um gefilterte Werte zu bestimmen, wobei der Betrieb des Filters in der Lage ist, abweichende Rohwerte zu eliminieren, welche aufgrund eines akustischen Störphänomens vorliegen können,
   c) - eine Berechnung einer Varianz (VAR), welche auf wenigstens einen Teil der gefilterten Werte durchgeführt wird, um durch Vergleich der Varianz mit einem Schwellenwert (S) eine Bedingung zum Berücksichtigen von Höhenunterschieden zu bestimmen, welche den festgestellten Unterschieden des Drucks oder der Höhe entsprechen,

d) - Berechnen des durch den Benutzer überwundenen Höhenunterschieds während seiner Fortbewegung, insbesondere den summierten positiven Höhenunterschied, mittels eines ersten Zählers (C1), welcher dazu vorgesehen ist, die Summe der überwundenen positiven Höhenunterschiede zu speichern, wobei die überwundenen positiven Höhenunterschiede berücksichtigt werden, wenn die Bedingung zum Berücksichtigen erfüllt ist.

2. Verfahren nach Anspruch 1, wobei der Betrieb des Filters einen Medianfilter verwendet, welcher auf eine vorbestimmte Anzahl von N der letzten Rohwerte wirkt, und der Betrieb des Filters im Takt der ersten Aufnahmefrequenz (F1) durchgeführt wird.

3. Verfahren nach einem der Ansprüche 1 und 2, wobei die Berechnung der Varianz bei einer zweiten Frequenz (F2) veranlasst wird, welche kleiner oder gleich der ersten ist.

4. Verfahren nach Anspruch 3, wobei die erste Frequenz (F1) ein Vielfaches der zweiten Frequenz (F2) ist, nämlich F1 = k F2.

5. Verfahren nach einem der Ansprüche 1 bis 4, wobei die Berechnung der Varianz auf P gefilterte unteraufgenommene Werte durchgeführt wird, welche in einem zweiten Puffer (B2) gespeichert sind.

6. Verfahren nach Anspruch 3, wobei die gefilterten Werte bei einer zweiten Frequenz unter-aufgenommen werden und die durch das Unter-Aufnehmen ausgewählten Werte in einem zweiten Puffer (B2) gespeichert werden.

7. Verfahren nach einem der Ansprüche 1 bis 5, ferner umfassend den folgenden Schritt:

e) - wenn das Ergebnis der Berechnung der Varianz VAR > Schwellenwert S, dann ist die Zählbedingung "erfüllt" und die festgestellten Höhenunterschiede werden durch die Berechnung der Schätzung des Höhenunterschieds berücksichtigt,

- wenn das Ergebnis der Berechnung der Varianz VAR < Schwellenwert S, dann ist die Zählbedingung "nicht erfüllt" und die festgestellten Höhenunterschiede werden durch die Berechnung der Schätzung des Höhenunterschieds nicht berücksichtigt.

8. Verfahren nach Anspruch 7, wobei während die Zählbedingung von "nicht erfüllt" zu "erfüllt" übergeht, der aufgenommene Höhenunterschied auf dem ältesten in dem zweiten Puffer (B2) gespeicherten Wert basiert.

9. Verfahren nach Anspruch 7, wobei beim ersten Mal, dass die Varianz VAR den Schwellenwert (S) übersteigt, der Höhenunterschied durch Berechnen der Differenz zwischen dem letzten in dem zweiten Puffer (B2) gespeicherten Wert und dem ältesten in dem zweiten Puffer (B2) gespeicherten Wert geschätzt wird.

10. Verfahren nach Anspruch 1, wobei wenn die Differenzen der festgestellten Höhe negativ sind, der Höhenunterschied einem Abstieg entspricht und in einem zweiten Zähler (C2) gespeichert wird.

11. Verfahren nach einem der Ansprüche 1 bis 5, **dadurch gekennzeichnet, dass** es in der Lage ist, einen Benutzer über seine physische Aktivität zu informieren, einschließlich dem Gehen zu Fuß, dem Rennen, dem Schlafen, dem Aufstieg des Gehens.

12. Verfahren nach einem der Ansprüche 1 bis 5, wobei ferner ein Beschleunigungssensor verwendet wird und wobei die Beschleunigungsinformationen verwendet werden, um die tatsächlichen Höhenunterschiede, welche mit einem Aufstieg verbunden sind, von denjenigen zu unterscheiden, welche mit einem mechanischen Aufstieg verbunden sind, wie beispielsweise mit einem Aufzug.

13. Vorrichtung, welche dazu eingerichtet ist, das Verfahren nach einem der Ansprüche 1 bis 12 zu implementieren.

14. Vorrichtung nach Anspruch 13, welche die Form eines Anzeigengehäuses für die persönliche Aktivität annimmt, welches in der Lage ist, einen Benutzer einer solchen Anzeige für die Aktivität über seine physische Aktivität zu informieren.

15. Vorrichtung nach Anspruch 13, welche einen integralen Teil eines Smartphones bildet.

**Claims**

1. Method for estimating the elevation change traveled by a user, said method using at least one pressure sensor adapted to provide raw pressure values and a temperature sensor adapted to provide temperature values in order to adjust the pressure values, said pressure sensor being adapted to be substantially held fixed to at least a portion of the body of said user during the implementation of said method, said method comprising at least:

a) - an operation of periodically sampling raw pressure values, reading the current raw pressure at a first sampling frequency (F1), the raw values being stored in the form of pressure or the corresponding altitude in a first buffer (B1),
b) - a filtering operation based on the raw values stored in the first buffer (B1) in order to determine filtered values, said filtering operation being able to eliminate aberrant raw values which can be caused by a disturbing acoustics phenomenon,
c) - a variance calculation (VAR), performed on at least a portion of the filtered values, in order to determine, by comparing said variance with a threshold (S), a condition for including elevation changes corresponding to the observed pressure or altitude differences,
d) - calculating the elevation change traveled by the user during his displacement, including the total positive elevation change, thanks to a first counter (C1), for storing the total of the positive elevation changes traveled, the positive elevation changes traveled being taken into account if the condition for taking into account is true.

2. Method according to claim 1 for which the filtering operation uses a median filter operating on a predefined number of N of the most recent raw values and the filtering operation is carried out at the rate of the first sampling frequency (F1).

3. Method according to any of claims 1 and 2 wherein the variance calculation is performed at a second frequency (F2), less than or equal to the first.

4. Method according to claim 3, for which the first frequency (F1) is a multiple of the second frequency (F2), namely F1 = k F2.

5. Method according to any of claims 1 to 4 for which the variance calculation is performed on P under-sampled filtered values, stored in a second buffer (B2).

6. Method according to claim 3, wherein the filtered values are under-sampled at the second frequency (F2), and the values selected by the under-sampling are stored in a second buffer (B2).

7. Method according to any of claims 1 to 5 further comprising the following step:

- if the result of the variance calculation VAR > threshold S then the condition for counting is 'true' and the elevation changes observed are taken into account by the calculation of the estimation of the elevation change
- if the result of the variance calculation VAR < threshold S then the condition for counting is 'false' and the elevation changes observed are not taken into account by the calculation of the estimation of the elevation change.

8. Method according to claim 7 for which when the condition for counting changes from 'false' to 'true', the elevation change taken into account is based on the oldest value stored in the second buffer (B2).

9. Method according to claim 7, wherein, the first time that the variance VAR exceeds the threshold (S), the elevation change is estimated by calculating the difference between the last value stored in the second buffer (B2), and the oldest value stored in the second buffer (B2).

10. Method according to claim 1, wherein if the differences in altitude observed are negative, the elevation change corresponds to a descent and is stored in a second counter (C2).

11. Method according to any of claims 1 to 5, **characterised in that** it is capable of informing a user about his physical activity including walking, running, sleep, stair climbing.

12. Method according to any of claims 1 to 5, further using an acceleration sensor and wherein the acceleration information is used to differentiate the elevation changes actually related to an ascent from those related to a mechanical ascent such as a lift.

13. Device adapted to implement the method according to any of claims 1 to 12.

14. Device according to claim 13 that takes the form of a personal activity monitor case capable of informing a user of such an activity monitor on his physical activity.

15. Device according to claim 13 which is an integral part of a smartphone.

# FIG. 1

# FIG. 2

# FIG. 3

# FIG. 4

# FIG. 5

## FIG. 6

## FIG. 7

**EP 2 870 911 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 5058427 A
- US 2012084053 A